# EUROPEAN PATENT APPLICATION

(11) **EP 3 249 407 A1**
(43) Date of publication of application: **29.11.2017**
(21) Application number: 17172988.2
(22) Date of filing: 26.05.2017
(51) Int. Cl.: G01N 33/569

(54) **METHOD FOR DETERMINING HERPES B VIRUS AND ASSAY SYSTEM THEREFORE**

(30) Priority: 26.05.2016 EP 16171518
(71) Applicant: Helmholtz-Zentrum für Infektionsforschung GmbH, 38124 Braunschweig (DE)
(72) Inventor: HOTOP, Sven-Kevin, 38312 Klein-Flöthe (DE); ABD EL WAHED, Ahmed, 37075 Göttingen (DE); BRÖNSTRUP, Mark, 38102 Braunschweig (DE); BEUTLING, Ulrike, 38124 Braunschweig (DE); SIEVERS, Claudia, 49685 Emstek (DE); CZERNY, Florian, 37077 Göttingen (DE); DIEDERICHSEN, Ulf, 37077 Göttingen (DE); CZERNY, Claus-Peter, 37136 Waake-Bösinghausen (DE); STAHL-HENNING, Christiane, 37083 Göttingen (DE); FRITZ, Hans-Joachim, 37120 Bovenden (DE); HUNSMANN, Gerhard, 37077 Göttingen (DE); FRANK, Ronald, 13187 Berlin (DE)
(74) Representative: Gramm, Lins & Partner Patent- und Rechtsanwälte PartGmbB

(57) **Abstract**

In a first aspect, the present invention relates to a method for determining the presence of herpes B virus, or for determining the status of herpes B virus infection in primates based on detection of antibodies against herpes B virus. In particular, the present invention relates to a method wherein at least two different peptides of short amino acid sequences with 4 to 40 amino acid residues present in the herpes B virus glycoproteins B, C, D, G, H and/or L are detected representing target regions of antibodies being specific against herpes B virus allowing determination of the presence of herpes B virus or determining the status of the herpes B virus infection in primates based on the presence and/or amount of the antibodies accordingly. In a further aspect, the present invention relates to an assay system for determining the presence of herpes B virus comprising at least said two different peptides derived from the herpes B virus glycoproteins B, C, D, G, H and/or L representing target regions of antibodies being specific against herpes B virus as well as means for determining the presence of said antibodies binding specifically to said peptides. Finally, the present invention relates to the use of at least two peptides comprising short amino acid sequences with 4 to 40 amino acids containing at least two different epitopes derived from herpes B glycoproteins B, C, D, G, H and/or L for determining the presence of herpes B virus or for determining the status of herpes B virus infection in primates.

## Description

In a first aspect, the present invention relates to a method for determining the presence of herpes B virus, or for determining the status of herpes B virus infection in primates based on detection of antibodies against herpes B virus. In particular, the present invention relates to a method wherein at least two different peptides of short amino acid sequences with 4 to 40 amino acid residues present in the herpes B virus glycoproteins B, C, D, G, H and/or L are detected representing target regions of antibodies being specific against herpes B virus allowing determination of the presence of herpes B virus or determining the status of the herpes B virus infection in primates based on the presence and/or amount of the antibodies accordingly. In a further aspect, the present invention relates to an assay system for determining the presence of herpes B virus comprising at least said two different peptides derived from the herpes B virus glycoproteins B, C, D, G, H and/or L representing target regions of antibodies being specific against herpes B virus as well as means for determining the presence of said antibodies binding specifically to said peptides. Finally, the present invention relates to the use of at least two peptides comprising short amino acid sequences with 4 to 40 amino acids containing at least two different epitopes derived from herpes B glycoproteins B, C, D, G, H and/or L for determining the presence of herpes B virus or for determining the status of herpes B virus infection in primates.

### Prior Art

Herpes B virus also known as *cercopithecine herpesvirus 1* or *herpesvirus simiae* also mentioned as *macacine herpesvirus 1* belongs to the genus simplex virus within the *Alphaherpesvirinae* subfamily of *Herpesviridae* family. Herpes B virus is endemic in many populations of macaques, both in the wild and in captivity. While in monkeys the virus elicits only mild clinical symptoms, if any, the virus can be transmitted by various routes to humans causing viral encephalitis with a high mortality rate. That is, while in the natural hosts, the virus exhibits pathogeneses similar to that of herpes simplex virus in humans, humans zoonotically infected with Herpes B virus have a severe central nervous system disease with a fatality rate of approximately 80%. Early diagnosis and subsequent treatment are crucial to human survival of the infection.

Due to the possibility of transmission by various routes, like bites, herpes B virus constitutes a considerable occupational hazard for animal caretakers, veterinarians and laboratory personnel as well as other people coming into contact with infected monkeys or other primates.

To reduce the risk of zoonotic infection as well as to establish and sustain maintenance of pathogen free monkey colonies, diagnosis and determination of herpes B virus is required. Among the measures envisaged are serological surveillance of monkey colonies and specific diagnosis of herpes B zoonosis against the background of antibodies recognizing the closely related humans herpes simplex virus HSV. Actually, in view of the close similarity to the herpes simplex virus (HSV) in human commercially available HSV tests have been used to determine herpes B virus infection in monkeys. The similarity at the level of genome organization between herpes B virus and human herpes simplex virus is on average approximately 62% identity of amino acid residues across all homologues proteins herpes B shares with both HSV-1 and HSV-2.

The herpes B virus genome was fully sequenced in 2003 from an isolate found in rhesus macaque. The viral envelope contains 12 glycoproteins, among them four with proven high immunogenicity, namely, glycoprotein B, glycoprotein C, glycoprotein D and membrane glycoprotein G.

Actually, glycoproteins D (gD) and B (gB) are major targets of the immune response in primates infected with herpes B virus. Antibodies against gB and gD are among the first to appear in serum during acute and recurrent HSV human infections and reach high titers that often persist for at least two years. Since gB is the most conserved envelope glycoprotein it is primarily responsible for extensive antigen cross-reactivity among the primate herpes virus.

Methods for detection of herpes B virus are described in US 5,487,969 B1 as well as in US 5,767,265 B1.

Therein, the sequence coding for the gB of herpes B virus is described.

Perelygina L., et al, J. Infect. Diseases, 2002, 186, 453-61 describe the identification of a herpes B virus-specific glycoprotein D immunodominant epitope recognized by natural and foreign hosts. Therein, an immunodominant gD peptide has been identified allegedly being useful as a type specific diagnostic antigen for detection of B virus antibodies in both macaque and human serum. The sequence described therein is at the very end of the C-terminus, namely, the peptide gD 362-370 present in the cytoplasmic tail of the glycoprotein D. However, it is also mentioned therein that absence of antibodies against the C-terminal end of the gD, namely, aa 362-370, was observed in several animals infected with herpes B virus. That is, although glycoprotein D contains immunodominant epitopes, in particular, the C-terminus, the C-terminus is not sufficient to establish a diagnostic method for herpes B virus accordingly.

Hotop, S-K., et al, PLOS One, 2014, 9(1), 1-11 disclose multiple antibody target regions on herpes B virus glycoproteins B and D identified by screening sera of infected rhesus macaques with peptide microarrays. Antibodies present in monkey sera that binds to substrates of the peptide collection were detected by microserological techniques. Actually, 18 different antibody target regions (ATRs) each may include a number of epitopes were recorded. The data provided therein allows determining the spatial distribution of herpes B virus epitopes within the glycoproteins B and D. It is speculated therein that profiling of sera at single epitope resolution may represent a powerful tool to comprehensively describe individual B-cell responses.

However, the prior art fails to provide a method and diagnostic tool for determining the presence of herpes B virus or for determining the status of herpes B virus infection in primates with sufficient sensitivity at high specificity, e.g. low rates of false positive and negative results. The commercially available HSV-based assays representing the prior art are not sufficient and sensitive enough to determine herpes B virus infection with high specificity and sensitivity.

Hence, it is an object of the present invention to provide a method allowing determination of the presence of herpes B virus or determination of the status of herpes B virus infection in primates with high specificity and sensitivity. Another object of the present invention is the provision of an assay system for determining the presence of herpes B virus or determining the status of herpes B virus infection with high sensitivity and high specificity.

### Brief description of the present invention

In a first aspect, the present invention relates to a method for determining the presence of herpes B virus, or for determining the status of herpes B virus infection in primates comprising the steps of
a) providing a sample, including providing a sample from a primate suspected to be infected with herpes B virus;
b) detecting specific antibodies in the sample against at least two different peptides of short amino acid sequences with 4 to 40 amino acid residues representing at least two different epitopes present in the herpes B virus glycoproteins B, C, D, G, H and/or L;
c) determining the presence of herpes B virus, or the status of herpes B virus infection in a primate based on the presence and/or on the amount of antibodies against at least two different epitopes present in the herpes B virus glycoproteins B, C, D, G, H and/or L, wherein the presence of said antibodies indicates the presence of the virus or wherein the presence and/or amount of the antibodies indicates the status of herpes B virus infection in said primate.

The inventors recognized that by detecting antibodies against at least two different epitopes present in the herpes B virus glycoproteins B, C, D, G, H and/or L, it is possible to determine the presence of herpes B virus or determining the status of herpes B virus infection in primates with higher specificity and sensitivity. The detection is based on detection of antibodies against herpes B virus. In a preferred embodiment, antibodies against at least two epitopes present on at least two different glycoproteins are determined. Typically, these epitopes are linear epitopes of synthetic peptides and not representing inactivated whole virus.

In a further aspect, the present invention relates to an assay system for determining the presence of herpes B virus comprising at least two different peptides derived from the herpes B virus glycoprotein B, C, D, G, H and/or L being antibody target regions of antibodies being specific against herpes B virus; means for determining the presence of said antibodies binding specifically to said peptides.

The assay system also identified as "assay" or "kit" according to the present invention represents a system allowing determination of herpes B virus with high specificity and sufficient sensitivity.

Finally, the present invention relates to the use of at least two peptides comprising short amino acid sequences with 4 to 40 amino acids containing at least two different epitopes derived from herpes B virus glycoproteins B, C, D, G, H and/or L.

### Brief description of the drawings

Figure 1: Overview of detected antibodies against 142 antibody target regions on 6 glycoproteins detected by peptide microarrays in 42 macaque serum samples. Protein length in aa is given as box. Each black bar represents a positive signal to the respective Region (ATR) and its relative position in the protein sequence.
Figure 2a: Distribution of detected antibodies against all ATRs on each glycoprotein and respective sequence length counted as spots.
Figure 2b: Relative immunogenicity of each antigen. Black bars were computed as possible target length (spots) and antibodies detected against respective ATRs (Hits).

### Detailed description of the present invention

In a first aspect, the present invention provides a method for determining the presence of herpes B virus, or for determining the status of herpes B virus infection in primates comprising the steps of
a) providing a sample, including providing a sample from a primate suspected to be infected with herpes B virus;
b) detecting specific antibodies against at least two different epitopes present in the herpes B virus glycoproteins B, C, D, G, H and/or L;
c) determining the presence of herpes B virus, or the status of herpes B virus infection in a primate based on the presence and/or on the amount of antibodies in the sample against at least two different epitopes present in herpes B virus glycoproteins B, C, D, G, H and/or L, wherein the presence of said antibodies indicates the presence of the virus or wherein the presence and/or amount of the antibodies indicates the status of herpes B virus infection in said primate.

The epitopes are present in peptides of short amino acid sequences with 4 to 40 amino acid residues representing antibody target regions (ATR).

The method according to the present invention allows determination of herpes B virus with higher sensitivity and higher specificity. That is, compared to the commercially available HSV-based method allowing detection of herpes B virus due to cross-reactivity, the method according to the present invention identifies false negative individuals in the HSV-test, thus, increasing sensitivity with high specificity. That is, the method according to the present invention is specific for herpes B virus while being non cross-reactive with HSV.

In this connection, the term "sensitivity" quantifies the avoiding of false negative while the term "specificity" quantifies the avoiding of false positives.

Since the herpes B virus is fatal in humans after zoonosis as well as to maintain pathogen-free primate colonies, high sensitivity of the method is required. As demonstrated herein, the method according to the present invention based on detecting specific antibodies against at least two different epitopes present in the herpes B virus glycoproteins B, C, D, G, H and/or L as defined increases sensitivity against herpes B virus.

In particular, the method is a serological method, however, the sample may represent any kind of sample obtained from body fluid or body tissue. The sample includes whole blood, plasma or serum as well as other body fluid sample or sample of the body fluid.

In the context of the present invention, the term "body fluid sample" or "sample of the body fluid" is a biological sample isolated from the subject which can include, without being limited thereto, whole blood, serum and plasma as well as urine, sputum, salvia, synovial, liquor. Preferably, the body fluid is serum or plasma. However, samples useful and methods according to the present invention for determining herpes B virus can be obtained also from tissue samples including biopsies, swab specimen from mucous membranes, or fine needle aspirates, or fresh or frozen tissue.

The term "determining" as used herein refers to assessing the presence, absence, quantity, level or amount of either a given substance within a clinical or subject derived sample. In particular, the term "determining" refers to assess physically the level or amount of antibodies specific to herpes B virus.

The term "primates" refers to a mammal of the order primates including the two main groups *prosimians* and *anthropoids.* The *prosimians* include *lemurs, lorisis* and *allies* while the *anthropoids* include old world monkeys and new world monkeys, gibbons, orang utan, gorillas, chimpanzees as well as humans. The primates include particularly humans and macaques belonging to the old world monkeys, a natural host of herpes B virus.

As used herein, the term "comprise" or "comprising" as well as the terms "contain" or "containing" include the embodiments of "consist of" or "consisting of".

The term "peptide" as used herein refers to a peptide chain composed of amino acid residues linked by peptide bonds or peptide like bonds. Peptides are composed of single amino acid residues in a defined order. The peptide according to the present invention is a peptide of at least 4 amino acid residues, like a peptide containing at least ten amino acid residues. For example, the peptide according to the present invention is a peptide of 40 amino acid residues at most, like peptides having a length in between 20 and 40 amino acid residues. The peptides may be obtained by genetic engineering techniques or may be obtained by peptide synthesis known in the art.

The term "epitope" also known as antigenic determinant is a part of an antigen that is recognized by the immune system, specifically by antibodies. The epitope is a specific piece of the antigen to which an antibody binds. Epitopes may occur as conformational epitopes or linear epitopes. In the present case it is preferred that a linear epitope is present, whereby said linear epitope is formed by a continuous sequence of amino acid residues from the antigen. Peptides representing antibody targeting regions (ATRs) usually contain more than one epitope.

In an embodiment of the present invention, the epitopes are present in different ATRs of the same glycoprotein or different glycoproteins. For example, the epitopes are present in different ATRs described in Hotop et al, see above.

The term "providing a sample" may include obtaining physically a sample from a subject. The term may include isolation from the subject. Typically, the sample is obtained in advance and the method according to the present invention includes the use of said obtained and isolated sample previously isolated from the subject.

The phrase "status of herpes B virus infection in a primate" refers to the presence, absence, or level or amount of herpes B virus based on the presence of antibodies present in the primate. The term "presence of herpes B virus" refers generally to the detection of herpes B virus based on the presence of antibodies in a sample.

The glycoproteins B, C, D, G, H and/or L of herpes B virus include the glycoprotein B (gB) of accession number NP_851887, glycoprotein C (gC), accession number NP_851904, glycoprotein D (gD), accession number NP_851925, glycoprotein G (gG), accession number NP_851923, glycoprotein H (gH), accession number NP_851881 and glycoprotein L (gL), accession number NP_851860.

The sequences of said glycoproteins are as follows: SEQ ID NO. 1 corresponds to NP_851887 (gB), SEQ ID NO. 2 corresponds to NP_851904 (gC), SEQ ID NO. 3 corresponds to NP_851925 (gD), SEQ ID NO. 4 corresponds to NP_851923 (gG), SEQ ID NO. 5 corresponds to NP_851881 (gH) and SEQ ID NO. 6 corresponds to NP_851860 (gL).

The glycoproteins mentioned herein include naturally occurring sequence varieties of the sequences of SEQ ID Nos. 1 to 6.

In an embodiment of the present invention, the epitopes are present in linear peptides and not as part of whole virus, in particular, inactivated virus. That is, typically, the epitopes are present as linear epitopes. For example, the epitopes are present as peptides having a length of 4 to 40 amino acid residues at most, like being peptides having a length of from 10 to 40 amino acid residues, like 20 to 40 amino acid residues. Examples of useful peptides are shown in SEQ ID Nos.7 to 12. That is, in an embodiment of the present invention the antibodies to be determined are directed against an epitope present in a peptide of a peptide of SEQ ID NO. 7 to 12. SEQ ID Nos. 7 to12 represent ATRs from gB (7), gC (8,9), gD (10,11) and gH (12).

In an embodiment of the present invention, the method is for determining the presence of herpes B virus or determining the status of herpes B virus infection in primates wherein antibodies are detected against at least two epitopes present on at least two different glycoproteins. In another embodiment, the two different glycoproteins are selected from the combination of i) glycoprotein D and glycoprotein C, ii) glycoprotein D and glycoprotein B, iii) glycoprotein C and glycoprotein B, or iv) glycoprotein H and a second glycoprotein selected from glycoprotein C, D or B. That is, while a first epitope is present in the first glycoprotein, the at least one further epitope is present in a second glycoprotein.

Thus, the present inventors recognized that the sensitivity is particularly increased when different antibodies are detected binding to at least two epitopes derived from one or more, like two different glycoproteins. That is, the present inventors recognized that a combination of peptides or full proteins of two different glycoproteins and determining antibodies, namely at least two different antibodies, binding thereto increases sensitivity while having a high specificity. The combinations identified above are particularly useful according to the present invention.

In an embodiment, the at least two epitopes are derived from glycoprotein D or glycoprotein C, in particular, wherein one of the two epitopes is present in SEQ ID NO. 10 or 11 and the other epitope is present in SEQ ID NO. 8 or 9.

As mentioned, particularly useful epitopes are present in the sequences of SEQ ID Nos. 7 to 12.

In another embodiment, the method according to the present invention is a method wherein at least three or more epitopes of at least two different glycoproteins selected from glycoproteins B, C, D, G, H and/or L are used.

Typically, the methods according to the present invention are *in vitro* methods. The skilled person is well aware of useful immunological methods or immunological detection methods allowing analyzing the sample for the presence or absence of antibodies against herpes B virus. Suitable methods comprise the formation of immune complexes (also called primary immune complexes) of antibodies and epitope comprising peptides. The skilled person is well aware of suitable methods for determining these immune complexes. For example, these complexes can be detected by a second binding ligand that has a binding affinity for the antibody present in the sample, for example to the Fc region of the antibodies or having reactivity to the antibody to be detected, e.g. a peptide having the same epitope, thus, forming a double antigen ELISA. In these cases, the second binding ligand can be linked to a detectable label or marker molecule. Typically, the second binding ligand is an antibody which may thus be termed a secondary antibody. For example, the immune complexes are contacted with the label, secondary binding ligand or antibody under conditions effective and for a period of time sufficient to allow the formation of secondary immune complexes. The secondary immune complexes are then generally washed to remove any unbound labeled secondary antibodies or ligands and the remaining label in the secondary immune complex is then detected. The second binding ligand, such as an antibody, having binding activity for the immune complex may also be used to bind to the primary immune complexes. The second binding ligand may contain an enzyme capable of processing or a substrate detectable to a product and, hence, amplify a signal over time. After washing, the secondary immune complexes are contacted with substrate, permitting detection. Alternatively, comparative immune protection may be used. The skilled person is well aware of suitable methods.

For example, suitable methods include immunological methods like ELISA, Blot systems including DOT Blot, peptide array systems or bead-based multiplex immunoassay platforms. An example of the bead-based multiplex immunoassay platform is the system developed and commercialized by Luminex Corp. In case of an ELISA, the peptides containing the epitopes representing the binding partner of the antibodies to be determined are bound to a solid support, such as for example a column matrix or a well of a microtiter plate, a membrane, beads, dipsticks or the like. Alternatively, the support can be provided as a separate element of the assay system according to the present invention or of a test kit.

In another embodiment, the method is for determining the presence of herpes B virus, or for determining the status of herpes B virus infection in primates whereby the primates are macaques or human. The method according to the present invention is particularly useful for determining herpes B virus in macaques housed as macaque colonies, or humans suspected to be afflicted with a herpes B virus infection due to zoonotic infection.

In another aspect, the present invention relates to an assay system for determining the presence of herpes B virus comprising at least two different peptides derived from herpes B virus glycoprotein B, C, D, G, H and/or L being antibody targets of antibodies being specific against herpes B virus as well as means for determining the presence of said antibodies binding specifically to said peptides.

That is, the assay system according to the present invention which can also be denoted as a kit, allows to detect antibodies recognizing specifically herpes B virus, in particular, allowing the differentiation between herpes B virus infection and HSV-infection, e.g. in humans.

The at least two different peptides containing the two different epitopes may be provided as a single polypeptide comprising a linker element. However, typically the two peptides are not in form of a polypeptide or as a full protein or as part of the whole virus.

For example, these at least two different peptides representing immune reactive peptides are derived from at least two different herpes B virus glycoproteins selected from glycoprotein B, C, D, G, H and/or L. In an embodiment, the peptides are at least three or more peptides derived from at least two different herpes B virus glycoproteins selected from glycoproteins B, C, D, G, H and/or L. In an embodiment, the peptides are at least three, like four, five, and in particular, all six peptides of SEQ ID Nos. 7 to 12. The assay system according to the present invention for use in determining the presence of herpes B virus in a sample includes in addition means for determining the presence of said antibodies binding specifically to the peptides containing the epitopes recognized by said antibodies. Said means are for example a detection agent for the antibodies which may be an antibody, antibody fragment, etc. In addition, the assay system may comprise more than one detection agent. If required, the assay system further comprises substrate and further means for allowing reaction with an enzyme used as a label for the detecting agent which may be an antibody.

The immunodetection agents representing the means for the presence of the antibodies binding specifically to the peptides comprising the herpes B virus specific epitopes can include detectable labels that are associated with or linked to the given detecting agent, in particular, the detecting antibody. Detectable labels that are associated with or attached to a secondary binding ligand are also contemplated. Detectable labels include dyes, luminescent or chromophores, like fluorescent molecules, biotin, radiolabels or enzymes. Typical examples for suitable labels include commonly known fluorescent molecules, like rhodamine, fluorescein, green fluorescent proteins or luciferase, or alkaline phosphatase and horseradish peroxidase as examples for suitable enzymes. In another embodiment, beads which include magnetic beads may be provided. In an embodiment, these beads are beads labeled additionally with a pre-determined chromophore like a fluorescence dye. Suitable systems include a bead-based multiplex immunoassay platform like the platform obtainable from Luminex, the so called Luminex system. That is, the assay system according to the present invention may be an immunological assay system, in particular, an ELISA, a Blot based system, a peptide array system, in particular, a peptide microarray system, or a bead-based multiplex immunoassay platform.

Optionally, the assay system further comprises positive and negative controls for verifying the results obtained when using the assay system. The components of the assay system can be packaged either in an aqueous medium or lyophilized form and, in addition, the assay system comprises one or more containers allowing to conduct the detection accordingly. In addition, the assay system comprises the instruction for use of the same.

Moreover, the present invention relates to the use of at least two peptides containing at least two different epitopes whereby these peptides are derived from herpes B virus glycoproteins B, C, D, G, H and/or L. The epitope present on each of the peptides represents herpes B virus specific epitopes. In an embodiment, three or more peptides having at least three or more epitopes of at least two different glycoproteins selected from herpes B virus glycoproteins B, C, D, G, H and/or L is used. For example, at least three, like four, five, and in particular, all peptides of SEQ ID Nos. 7 to 12 are used for determining the presence of herpes B virus or for determining the status of herpes B virus infection in primates. The peptides useful for determination of the presence of herpes B virus or for determining the status of herpes B virus infection in primates contain at least one herpes B virus specific epitope. Said epitope is recognized by antibodies specific against herpes B virus. In particular, the epitopes allow to differentiate between HSV and herpes B virus. That is, the antibodies against HSV will not recognize and will not bind to the epitope present on said peptide.

The use according to the present invention allows to identify specifically herpes B virus.

As identified, the samples may be obtained from body fluid. In an embodiment of the present invention, a purification thereof is conducted in advance before detecting specific antibodies against the herpes B virus glycoproteins. That is, to reduce unspecific binding and to increase the detection limit, a purification of the serum samples may be useful. For example, pre-incubation of the serum samples to be analyzed using a cellulose-linker construct may be helpful to reduce cross reactivity (unspecific binding) and to detect low signals. Thereby any kind of cross reactivity to the linker-construct itself (i.e. other serum proteins like albumins or transferrin) will be reduced. Consistently low signals (antibody binding) to peptides will be detectable which otherwise would be hidden due to high background of unwanted bound material to the used cellulose-linker conjugate, as described in the Examples.

That is, it may be helpful for some methods that a pre-purification of the sample to be analyzed is conducted.

According to the present invention, the peptides may be modified e.g. by adding certain molecules to the C- or N-terminus that allow binding to a solid support. In the present approach, a PEG spacer composed of two-head-to-tail-linked 8-amino-3,6-dioxaoctanoic acid molecules was coupled to the N-terminus of the peptides. Biotin was coupled to the spacer group which allows the immobilization on the avidin-coated magnetic beads of the Luminex system.

Other modifications are possible to allow binding to the desired support accordingly.

The present invention will be described further by way of examples without limiting the same.

### Examples:

### Example 1

### Peptide Microarrays

Peptide-Microarray experiments were done as described before (1, Hotop S.-K. et al., see above), except the following changes were made to the respective protocols. Samples were pretreated as follows: Eppendorf-tubes were coated with 2mM of dissolved cellulose-membrane with a short peptide chain composed of two beta-alanine molecules (cellulose-linker conjugate) in 100mM Sodium-Carbonate Buffer (pH9.6) over night at 4°C. Afterwards tubes were washed 3 times with TBS followed by 3 times absolute ethanol and stored after drying at -20°C until usage. Tubes were treated in same manner as described earlier for handling of slides (1), allowing unspecific binding of any component to cellulose-linker conjugate (cross-reactivity) in order to obtain purification of primary antibodies. In brief, samples were diluted 1:120 in blocking buffer (2% Casein in TBS-T) and binding was carried out over night at 4°C. Afterwards samples were transferred into a chamber system (100µl each chamber, HybriWell™ sealing system, Grace Bio Labs, USA, Oregon) attached to the surface of previously treated and blocked microarray slides and incubated as described earlier (1).

### Chip-Layout:

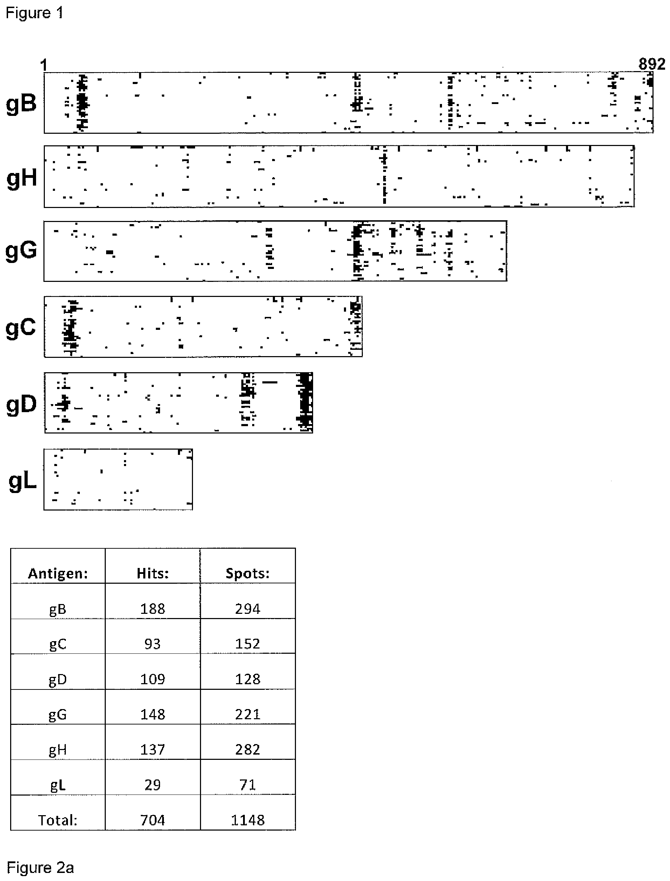

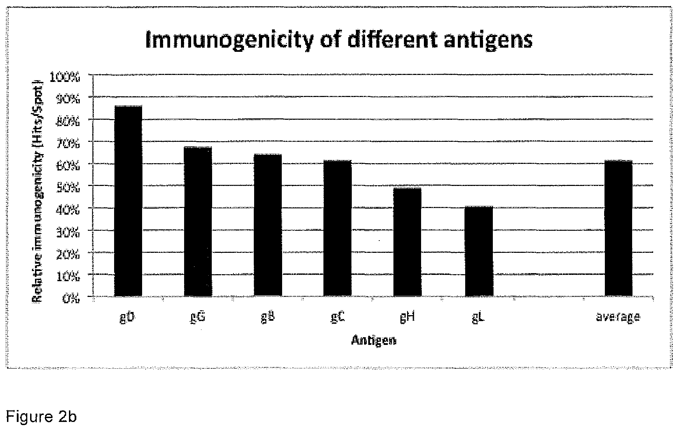

To address the question, whether more epitopes on ATRs can bring down false negative rates of the first study down to <000.1%, 4 new major antigens of proven high immunogenicity on herpes B viruses (gC (NP_851904), gG (NP_851923), gH (NP_851881) and gL (NP_851860)) as well as both gB (NP_851887) and gD (NP_851925) used in the first study, were synthesized as 15mer overlapping peptides with an offset of 3 amino acids as described before (1). In addition, 24 randomly obtained sequences of 15mers were also spotted to each region on the slide suitable for a chamber created by attaching the HybriWell™ sealing system to the slide surface, allowing determination of unspecific binding, resulting in 4688 peptides per slide and 1172 peptides in duplicates per chamber for screening two individual samples per slide. An overview of the data obtained is shown in figures 1 and 2a and 2b.

Further, table 1 shows the data of 42 samples obtained from macaques tested.

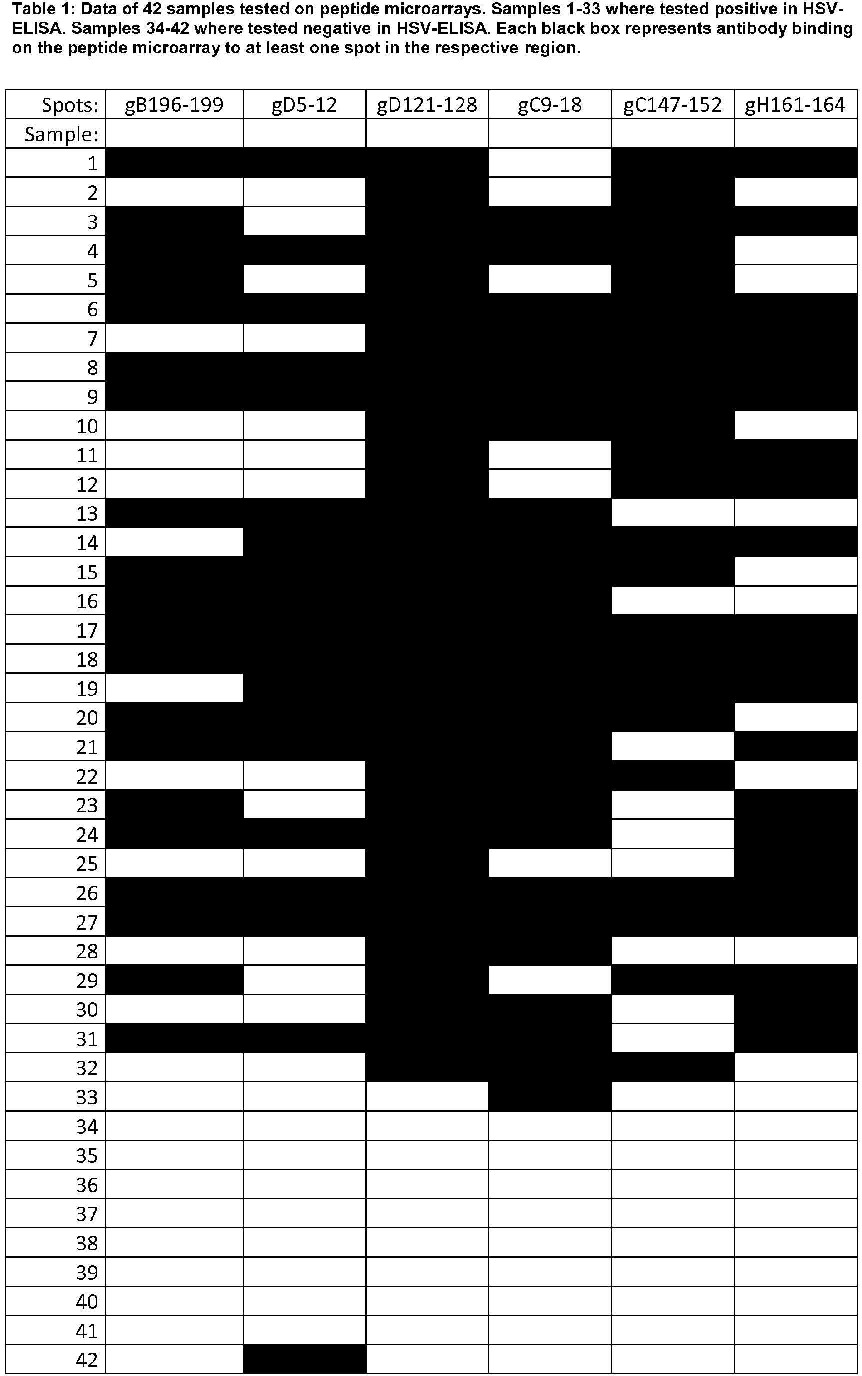

## Claims

1. A method for determining the presence of herpes B virus, or for determining the status of herpes B virus infection in primates comprising the steps of
a) providing a sample, including providing a sample from a primate suspected to be infected with herpes B virus;
b) detecting specific antibodies in the sample against at least two different epitopes present in the herpes B virus glycoproteins B, C, D, G, H and/or L;
c) determining the presence of herpes B virus, or the status of herpes B virus infection in a primate based on the presence and/or on the amount of antibodies against at least two different epitopes present in the herpes B virus glycoproteins B, C, D, G, H and/or L, wherein the presence of said antibodies indicates the presence of the virus or wherein the presence and/or amount of the antibodies indicates the status of herpes B virus infection in said primate.

2. The method for determining the presence of herpes B virus or determining the status of herpes B virus infection in primates wherein the antibodies are directed against epitopes present in at least two different glycoproteins.

3. The method according to claim 2 wherein the two different glycoproteins are selected from the combination of i) glycoprotein D and glycoprotein C, ii) glycoprotein D and glycoprotein B, iii) glycoprotein C and glycoprotein B, or iv) glycoprotein H and a second glycoprotein selected from glycoprotein C, D or B.

4. The method according to claim 1 wherein the at least two epitopes are derived from glycoprotein D and/or glycoprotein C, in particular, wherein one of the two epitopes is present in SEQ ID NO. 10 or 11 and the other epitope is present in SEQ ID NO. 8 or 9.

5. The method according to any one of the preceding claims wherein at least three or more epitopes of at least two different glycoproteins selected from glycoproteins B, C, D, G, H and/or L are used.

6. The method according to any one of the preceding claims wherein the epitopes are present as peptides having a length of from 4 to 40 amino acid residues at most, preferably, having a length of from 20 to 40 amino acid residues.

7. The method according to any one of the preceding claims wherein the epitopes detected are present in a peptide of at least two, preferably at least three of the sequences of SEQ ID Nos. 7 to 12.

8. The method according to any one of the preceding claims being an immunological method, in particular, an ELISA, a peptide array system, a Blot system, or a bead-based multiplex immunoassay platform.

9. The method according to any one of the preceding claims wherein the primates are macaque or human.

10. An assay system for determining the presence of herpes B virus comprising at least two different peptides derived from the herpes B virus glycoprotein B, C, D, G, H and/or L being target regions of antibodies being specific against herpes B virus; means for determining the presence of said antibodies binding specifically to said peptides.

11. The assay system according to claim 10 wherein said peptides are at least two peptides derived from at least two different herpes B virus glycoproteins selected from glycoprotein B, C, D, G, H and/or L.

12. The assay system according to claim 10 or 11 wherein at least three or more peptides of at least two different herpes B virus glycoproteins selected from glycoproteins B, C, D, G, H and/or L are present.

13. The assay system according to claims 10 to 12 wherein said peptides are at least three, like four, five, and, in particular, all peptides of SEQ ID Nos. 7 to 12.

14. The assay system according to any one of claims 10 to 13 being an immunological assay system, in particular, an ELISA, a Blot based system, a peptide array system, in particular, a peptide microarray system, or a bead-based multiplex immunoassay platform.

15. The use of at least two peptides containing at least two different epitopes derived from herpes B virus glycoproteins B, C, D, G, H and/or L, in particular, three or more peptides having at least three or more epitopes of at least two different glycoproteins selected from herpes B virus glycoproteins B, C, D, G, H and/or L, like at least three, like four, five and, in particular, all peptides of SEQ ID Nos. 7 to 12 for determining the presence of herpes B virus or for determining the status of herpes B virus infection in primates.


SEQUENCE LISTING

<110>  Helmholtz-Zentrum fuer Infektionsforschung GmbH

<120>  Method for determining herpes B virus and assay system therefore

<130>  3275-042-EP-1

<150>  EP16171518
<151>  2016-05-26

<160>  12

<170>  PatentIn version 3.5

<210>  1
<211>  892
<212>  PRT
<213>  Herpes B virus

<400>  1

Met Arg Pro Arg Ala Gly Pro Leu Pro Leu Pro Ser Pro Leu Val Pro
1               5                   10                  15

Leu Leu Ala Leu Ala Leu Leu Ala Ala Thr Arg Pro Leu Gly Pro Ala
            20                  25                  30

Ala Ala Thr Pro Val Val Ser Pro Arg Ala Ser Pro Ala Pro Pro Val
        35                  40                  45

Pro Ala Ala Thr Pro Thr Phe Pro Asp Asp Asp Asn Asp Gly Glu Ala
    50                  55                  60

Gly Ala Ala Pro Gly Ala Pro Gly Thr Asn Ala Ser Val Glu Ala Gly
65                  70                  75                  80

His Ala Thr Leu Arg Glu Asn Leu Arg Asp Ile Lys Ala Leu Asp Gly
                85                  90                  95

Asp Ala Thr Phe Tyr Val Cys Pro Pro Pro Thr Gly Ala Thr Val Val
            100                 105                 110

Gln Phe Glu Gln Pro Arg Pro Cys Pro Arg Ala Pro Asp Gly Gln Asn
        115                 120                 125

Tyr Thr Glu Gly Ile Ala Val Ile Phe Lys Glu Asn Ile Ala Pro Tyr
    130                 135                 140

Lys Phe Lys Ala Thr Met Tyr Tyr Lys Asp Val Thr Val Ser Gln Val
145                 150                 155                 160

Trp Phe Gly His Arg Tyr Ser Gln Phe Met Gly Ile Phe Glu Asp Arg

                165                 170                 175

Ala Pro Val Pro Phe Glu Glu Val Ile Asp Lys Ile Asn Ala Arg Gly
            180                 185                 190

Val Cys Arg Ser Thr Ala Lys Tyr Val Arg Asn Asn Met Glu Ser Thr
        195                 200                 205

Ala Phe His Arg Asp Asp Asp Glu Ser Asp Met Lys Leu Lys Pro Ala
    210                 215                 220

Lys Ala Ala Thr Arg Thr Ser Arg Gly Trp His Thr Thr Asp Leu Lys
225                 230                 235                 240

Tyr Asn Pro Ser Arg Ile Glu Ala Phe His Arg Tyr Gly Thr Thr Val
                245                 250                 255

Asn Cys Ile Val Glu Glu Val Glu Ala Arg Ser Val Tyr Pro Tyr Asp
            260                 265                 270

Glu Phe Val Leu Ala Thr Gly Asp Phe Val Tyr Met Ser Pro Phe Tyr
        275                 280                 285

Gly Tyr Arg Asp Gly Ala His Ala Glu His Thr Ala Tyr Ala Ala Asp
    290                 295                 300

Arg Phe Arg Gln Val Asp Gly Tyr Tyr Glu Arg Asp Leu Ser Thr Gly
305                 310                 315                 320

Arg Arg Ala Ser Thr Pro Ala Thr Arg Asn Leu Leu Thr Thr Pro Lys
                325                 330                 335

Phe Thr Val Gly Trp Asp Trp Ala Pro Lys Arg Pro Ser Val Cys Thr
            340                 345                 350

Leu Thr Lys Trp Gln Glu Val Asp Glu Met Leu Arg Ala Glu Tyr Gly
        355                 360                 365

Pro Ser Phe Arg Phe Ser Ser Ser Ala Leu Ser Thr Thr Phe Thr Thr
    370                 375                 380

Asn Arg Thr Glu Tyr Ala Leu Ser Arg Val Asp Leu Gly Asp Cys Val
385                 390                 395                 400

Gly Arg Glu Ala Arg Glu Ala Val Asp Arg Ile Phe Leu Arg Arg Tyr
                405                 410                 415

Asn Gly Thr His Val Lys Val Gly Gln Val Gln Tyr Tyr Leu Ala Thr
            420                 425                 430

Gly Gly Phe Leu Ile Ala Tyr Gln Pro Leu Leu Ser Asn Ala Leu Val
        435                 440                 445

Glu Leu Tyr Val Arg Glu Leu Leu Arg Glu Gln Glu Arg Arg Pro Gly
    450                 455                 460

Asp Ala Ala Ala Thr Pro Lys Pro Ser Ala Asp Pro Pro Asp Val Glu
465                 470                 475                 480

Arg Ile Lys Thr Thr Ser Ser Val Glu Phe Ala Arg Leu Gln Phe Thr
                485                 490                 495

Tyr Asp His Ile Gln Arg His Val Asn Asp Met Leu Gly Arg Ile Ala
            500                 505                 510

Ile Ala Trp Cys Glu Leu Gln Asn His Glu Leu Thr Leu Trp Asn Glu
        515                 520                 525

Ala Arg Lys Leu Asn Pro Asn Ala Ile Ala Ser Ala Thr Val Gly Arg
    530                 535                 540

Arg Val Ser Ala Arg Met Leu Gly Asp Val Met Ala Val Ser Thr Cys
545                 550                 555                 560

Val Pro Val Thr Pro Asp Asn Val Ile Met Gln Asn Ser Met Arg Val
                565                 570                 575

Pro Ala Arg Pro Gly Thr Cys Tyr Ser Arg Pro Leu Val Ser Phe Arg
            580                 585                 590

Tyr Glu Glu Gly Gly Pro Leu Val Glu Gly Gln Leu Gly Glu Asp Asn
        595                 600                 605

Glu Ile Arg Leu Glu Arg Asp Ala Leu Glu Pro Cys Thr Val Gly His
    610                 615                 620

Arg Arg Tyr Phe Thr Phe Gly Ala Gly Tyr Val Tyr Phe Glu Asp Tyr
625                 630                 635                 640

Ala Tyr Ser His Gln Leu Gly Arg Ala Asp Val Thr Thr Val Ser Thr
                645                 650                 655

Phe Ile Asn Leu Asn Leu Thr Met Leu Glu Asp His Glu Phe Val Pro
            660                 665                 670

Leu Glu Val Tyr Thr Arg Gln Glu Ile Lys Asp Ser Gly Leu Leu Asp
        675                 680                 685

Tyr Thr Glu Val Gln Arg Arg Asn Gln Leu His Ala Leu Arg Phe Ala
    690                 695                 700

Asp Ile Asp Thr Val Ile Lys Ala Asp Ala His Ala Ala Leu Phe Ala
705                 710                 715                 720

Gly Leu Tyr Ser Phe Phe Glu Gly Leu Gly Asp Val Gly Arg Ala Val
                725                 730                 735

Gly Lys Val Val Met Gly Ile Val Gly Gly Val Val Ser Ala Val Ser
            740                 745                 750

Gly Val Ser Ser Phe Leu Ser Asn Pro Phe Gly Ala Leu Ala Val Gly
        755                 760                 765

Leu Leu Val Leu Ala Gly Leu Ala Ala Ala Phe Phe Ala Phe Arg Tyr
    770                 775                 780

Val Met Arg Leu Gln Arg Asn Pro Met Lys Ala Leu Tyr Pro Leu Thr
785                 790                 795                 800

Thr Lys Glu Leu Lys Ser Asp Gly Pro Ser Pro Ala Gly Asp Gly Gly
                805                 810                 815

Asp Gly Ala Ser Gly Gly Gly Glu Glu Asp Phe Asp Glu Ala Lys Leu
            820                 825                 830

Ala Gln Ala Arg Glu Met Ile Arg Tyr Met Ala Leu Val Ser Ala Met
        835                 840                 845

Glu Arg Thr Glu His Lys Ala Arg Lys Lys Gly Thr Ser Ala Leu Leu
    850                 855                 860

Ser Ala Lys Val Thr Asn Met Val Met Arg Lys Arg Ala Lys Pro Arg
865                 870                 875                 880

Tyr Ser Pro Leu Gly Asp Thr Asp Glu Glu Glu Leu
                885                 890

<210>  2
<211>  467
<212>  PRT
<213>  Herpes B virus

<400>  2

Met Ala Gly Trp Arg Ala Ala Gly Ser Gly Leu Cys Leu Phe Val Leu
1               5                   10                  15

Met Trp Leu Leu Cys Ala Gly Ala Gly Ala Pro Arg Gly Ala Ala Ser
            20                  25                  30

Thr Pro Ala Gly Arg Pro Gly Ala Ser Arg Pro Gly Gly Val Glu Arg
        35                  40                  45

Ala Asn Arg Thr Ala Ala Pro Ala Arg Gly Arg Gly Ser Ser Asn Gly
    50                  55                  60

Thr Gly Pro Gly Ser Thr Ser Ala Gln Phe Arg Cys Lys Arg Pro Asp
65                  70                  75                  80

Val Ser Ala Leu Tyr Gly Ser Arg Val Val Ile Gly Cys Arg Leu Pro
                85                  90                  95

Arg Pro Thr Ala Asp Phe Arg Leu Gln Ile Trp Arg Val Ala Ala Ala
            100                 105                 110

Ala His Thr Glu Pro Val Glu Pro Gly Ala Val Leu Val Asn Val Thr
        115                 120                 125

Ala Pro Pro Asp Gly Glu Leu Val Tyr Asp Ser Ala Pro Asn Arg Thr
    130                 135                 140

Glu Ala Arg Val Arg Trp Ala Glu Gly Ala Gly Pro Asp Ala Arg Pro
145                 150                 155                 160

Arg Val Tyr Ser Ile Glu Gly Thr Phe Pro Thr Gln Arg Leu Val Ile
                165                 170                 175

Gln Glu Leu Thr Val Ala Arg Gln Gly Leu Tyr Leu Trp Ile Arg Gly
            180                 185                 190

Pro Ala Glu Arg Pro Leu Arg Tyr Gly Thr Trp Thr Arg Val Arg Met
        195                 200                 205

Leu Arg Arg Pro Ser Leu Ser Ile Arg Ala His Thr Val Leu Glu Gly
    210                 215                 220

Glu Pro Phe Gly Ala Thr Cys Val Ala Ala Asn Tyr Tyr Pro Gly Asp
225                 230                 235                 240

Arg Ala Ala Phe Arg Trp Phe Glu Gly Gly Gly Glu Val Val Ala Pro
                245                 250                 255

Glu Arg Val Gln Thr Arg Val Asp Ala Gln Arg Asn Gly Phe Ser Ala
            260                 265                 270

Thr Ser Thr Leu Thr Ser Glu Ala Arg Ala Gly Leu Ala Pro Pro Arg
        275                 280                 285

Asn Leu Thr Cys Glu Phe Thr Trp His Arg Asp Ser Val Ser Phe Ser
    290                 295                 300

Arg Arg Asn Ala Thr Gly Ala Pro Thr Val Leu Pro Arg Pro Thr Ile
305                 310                 315                 320

Glu Met Glu Phe Gly Ser Gly Glu Ala Val Cys Thr Ala Ala Cys Val
                325                 330                 335

Pro Glu Gly Val Glu Leu Gln Trp Leu Leu Gly Ala Asp Pro Ala Pro
            340                 345                 350

Ala Glu Asp Ala Ala Ala Ser Gly Gly Pro Cys Pro Gly His Pro Gly
        355                 360                 365

Leu Ala Arg Val Arg Ser Ala Leu Pro Leu Ser Arg Glu His Ser Glu
    370                 375                 380

Tyr Thr Cys Arg Leu Val Gly Tyr Pro Pro Thr Val Pro Val Leu Glu
385                 390                 395                 400

His His Gly Arg His Glu Pro Ala Pro Arg Asp Pro Val Gly Gln Gln
                405                 410                 415

Val Thr Thr Ala Leu Glu Trp Ala Gly Ile Ala Ala Gly Ser Ala Ala
            420                 425                 430

Ala Ile Gly Leu Ala Val Gly Val Gly Val Tyr Val Arg Arg Ala Val
        435                 440                 445

Ala Arg Arg Arg Arg Val Arg Thr Gly Arg Trp Ala Gly Glu Pro Ala
    450                 455                 460

Arg Arg Gly
465

<210>  3
<211>  394
<212>  PRT
<213>  Herpes B Virus

<400>  3

Met Gly Pro Gly Ile Ala Ala Val Leu Leu Ser Leu Ala Val Ala Leu
1               5                   10                  15

Ala Arg Val Pro Ala Gly Gly Gly Glu Tyr Val Pro Val Glu Arg Ser
            20                  25                  30

Leu Thr Arg Val Asn Pro Gly Arg Phe Arg Gly Ala His Leu Ala Pro
        35                  40                  45

Leu Glu Gln Lys Thr Asp Pro Pro Asp Val Arg Arg Val Tyr His Val
    50                  55                  60

Gln Pro Phe Val Glu Asn Pro Phe Gln Thr Pro Ser Val Pro Val Ala
65                  70                  75                  80

Val Tyr Tyr Ala Val Leu Glu Arg Ala Cys Arg Ser Val Leu Leu Trp
                85                  90                  95

Ala Pro Thr Glu Ala Val Gln Val Val Arg Gly Ala Pro Glu Ala Thr
            100                 105                 110

Arg Pro Asp Ala Arg Tyr Asn Leu Thr Val Ala Trp Tyr Arg Thr Ser
        115                 120                 125

Asp Asp Cys Ala Ile Pro Ile Leu Val Met Glu Tyr Ala Glu Cys Pro
    130                 135                 140

Tyr Asp Arg Pro Leu Gly Ala Cys Pro Val Arg Asn Leu Pro Arg Trp
145                 150                 155                 160

Ser Phe Tyr Asp Asn Phe Gly Ala Thr Ser Asp Asp Asp Leu Gly Leu
                165                 170                 175

Val Met His Ala Pro Ala Phe Glu Thr Ala Gly Thr Tyr Val Arg Leu
            180                 185                 190

Val Lys Val Asn Gly Trp Val Glu Val Thr Gln Phe Ile Phe Glu His
        195                 200                 205

Arg Gly Lys Gly Pro Cys Arg Tyr Thr Leu Pro Leu Arg Ile Leu Pro
    210                 215                 220

Ala Ala Cys Leu Arg Gly Pro Val Phe Glu Gln Gly Val Thr Val Asp
225                 230                 235                 240

Gly Ile Gly Met Leu Pro Arg Phe Ile Pro Glu Asn Gln Arg Ile Val

245                 250                 255

Ala Val Tyr Ser Leu Gln Ala Ala Gly Trp His Gly Pro Lys Ala Pro
            260                 265                 270

Phe Thr Ser Thr Leu Leu Pro Pro Glu Val Val Glu Thr Ala Asn Ala
        275                 280                 285

Thr Arg Pro Glu Leu Ala Pro Glu Asp Glu Asp Glu Gln Ala Pro Gly
    290                 295                 300

Asp Glu Pro Ala Pro Ala Val Ala Ala Gln Leu Pro Pro Asn Trp His
305                 310                 315                 320

Val Pro Glu Ala Ser Asp Val Thr Ile Gln Gly Pro Ala Pro Ala Pro
                325                 330                 335

Ser Gly His Thr Gly Ala Ile Val Gly Ala Leu Ala Gly Ala Gly Leu
            340                 345                 350

Ala Ala Gly Val Val Val Leu Ala Val Tyr Leu Val Arg Arg Arg Ala
        355                 360                 365

Arg Ala Ala Gly Lys His Val Arg Leu Pro Glu Leu Leu Asp Glu Gly
    370                 375                 380

Pro Gly Pro Ala Arg Arg Gly Ala Pro Tyr
385                 390

<210>  4
<211>  673
<212>  PRT
<213>  Herpes B virus

<400>  4

Met Arg Arg Leu Val Pro Arg Leu Ser Leu Leu Ala Ala Val Val Cys
1               5                   10                  15

Leu Cys Leu Gly Pro Ala Gly Ala Gly Pro Arg Pro Pro Ala Ala Pro
            20                  25                  30

Pro Asp Ala Ala Pro Ala Ala Arg Asp Pro Ala Tyr Cys Tyr Ala Val
        35                  40                  45

Pro Arg Leu Asp Asp Val Gly Pro Ala Gly Pro Ala Pro Pro Val Ala
    50                  55                  60

Asp Val Asp Arg His Pro Val Val Arg His Arg Asn Leu Ser Arg Ser

65                  70                  75                  80

Tyr Ser Arg Arg Gly Leu Ala Leu Leu Val Pro Pro Ile Arg Arg Phe
                85                  90                  95

Arg Gly Thr Gly Gly Gly Ala Tyr Ala Ala Arg Val Ala Tyr Tyr Arg
            100                 105                 110

Val Asp Ala Gly Gly Cys Arg Arg Pro Ile Ala Leu Arg Trp Tyr Gly
        115                 120                 125

Gly Cys Arg Gly Gly Ala Pro Pro Ser Pro Ser Thr Cys Gly Arg Tyr
    130                 135                 140

Ser His Thr Tyr His Asp Gly Ser Pro Pro Met Ala His Ala Leu Val
145                 150                 155                 160

Asn Ala Ser Leu Leu Val Pro Val Val Gly Ser Arg Ala Ala Ala Tyr
                165                 170                 175

Asp Tyr Glu Ile Leu Val Gly Asp Arg Leu His Val Gly Arg Leu Thr
            180                 185                 190

Ala Ala Val Ala Pro Gly Asp Pro Leu Ser Pro Pro Asp Arg Ala Ser
        195                 200                 205

Arg Pro Asp Asp Arg Gly Arg Gly Gly Pro Ala Ala Cys Arg Pro Val
    210                 215                 220

Ala Pro Ala Val Pro Leu Trp Arg Ser Ile Pro Ala Ala Trp Val Val
225                 230                 235                 240

Ala Ala Asn Pro Leu Phe Asp Gly Pro Glu Gly Arg Arg Cys Val Ser
                245                 250                 255

Pro Gln Asp Pro Ala Val Pro Gln Asp Met Ala Tyr Val Ser Ala Ala
            260                 265                 270

Pro Gln Ser Leu Leu Val Gly Leu Ser Gly Tyr Leu Phe Ala Leu Gly
        275                 280                 285

Gly Arg Asp Arg Glu Gly Glu Pro Pro Ala Thr Glu Ala Pro Ala Pro
    290                 295                 300

Thr Ala Glu Arg Phe Ala Arg Ala Arg Leu Ala Arg Ser Leu Leu Ser
305                 310                 315                 320

Val Thr Thr Glu Gly Thr Glu Ala Ser Pro Ala Ser Ala Pro Thr Val
                325                 330                 335

Ala Pro Ser Ala Ala Thr Glu Gly Ala Ser Ala Thr Ser Gly Ser Ala
            340                 345                 350

Thr Ala Ala Ser Ala Ala Val Glu Ser Ala Thr Pro Thr Ala Asn Gly
        355                 360                 365

Thr Ala Ala Thr Pro Ala Pro Thr Thr Thr Pro Ala Pro Thr Thr Thr
    370                 375                 380

Pro Ala Pro Thr Thr Thr Pro Ala Pro Thr Thr Thr Pro Ala Pro Thr
385                 390                 395                 400

Thr Thr Pro Ala Pro Thr Thr Thr Pro Ala Asn Gly Thr Ala Gly Ala
                405                 410                 415

Glu Ser Asp Ala Gly Glu Gly Ser Lys Thr Thr Pro Ala Thr Pro Thr
            420                 425                 430

Ala Ala Pro Ser Val Ser Pro Ala Pro Ala Ala Pro Ala Ala Asn Asp
        435                 440                 445

Thr Gly Ala Ala Gly Ala Gly Pro Val Pro Ser Asp Ala Thr Thr Pro
    450                 455                 460

Pro Gly Pro Thr Ala Ala Glu Glu Asn Glu Ser Ser Pro Pro Leu Pro
465                 470                 475                 480

Arg Glu Gly Gly Gly Asp Ser Ala Thr Gly Gly Ala Ser Ala Ala Ala
                485                 490                 495

Gly Thr Glu Ala Pro Pro Ala Asp Pro Phe Thr Pro Arg Leu Glu Ala
            500                 505                 510

Leu Thr Pro Arg Pro Gly Ala Gly Gly Gly Ala Glu Arg Gly Ala Gly
        515                 520                 525

Glu Glu Asp Gly Asp Asp Asp Asp Asp Glu Glu Gly Ala Gly Asp Gly
    530                 535                 540

Glu Leu Pro Pro Ala Glu Asn Pro Pro Pro Ala Thr Gly His Ala Gly
545                 550                 555                 560

Pro Gly Ala Arg Pro Asp Ser His Pro Tyr Pro Pro Thr Pro Arg Arg
                565                 570                 575

Pro Ser Ser Arg Leu Pro Pro Pro His Leu Gly Pro Leu Thr Leu Arg
            580                 585                 590

Pro Pro Arg Pro Pro Thr Glu Pro Gln Ala Pro Ser Pro Ser Pro Thr
        595                 600                 605

Glu Pro Pro His Thr Pro Leu Phe Pro Phe Leu Thr Ala Ser Pro Gly
    610                 615                 620

Leu Asp Phe Leu Phe Leu Leu Ser Leu Ala Ala His Ala Leu Ala Phe
625                 630                 635                 640

Val Val Ile Thr Val Met Val Leu Arg Pro Cys Arg Pro Arg Ala Arg
                645                 650                 655

Ser Pro Ser Gln His Arg Val Arg Tyr Thr Arg Leu Pro Thr Ser His
            660                 665                 670

Ala

<210> 5
<211> 858
<212> PRT
<213> Herpes B virus

<400> 5

Met Ser Ala Arg Arg Arg Asp Arg Ser Thr Gly Met Pro Val Cys Trp
1               5                   10                  15

Ile Leu Ala Gly Leu Ala Ile Ala Ala Gly Ser Ala Ala Val Pro Ala
            20                  25                  30

Pro Met Arg Ala Leu Glu Arg Glu His Tyr Trp Val Ala Gln Ala Asp
        35                  40                  45

Ser Trp Tyr Arg Asp His Pro Arg Met Arg Ala Tyr Trp Arg Asp Gly
    50                  55                  60

Glu Pro Ser Arg Leu Trp Leu Pro Asn Leu Pro Asn Ala Thr Lys Leu
65                  70                  75                  80

Pro Leu Gly Leu Leu Ala Pro Pro Ala Glu Leu Asn Leu Thr Val Ala
                85                  90                  95

Thr Ala Pro Leu Leu Arg Trp Ala Thr Pro Arg Ser Cys Phe Leu Phe
            100                 105                 110

Ile Thr Thr Pro Glu Phe Pro Arg Asn Pro Gly Gln Leu Leu Tyr Val
        115                 120                 125

Asn Lys Thr Ala Leu Leu Gly Leu Pro Ala Asn Ala Ser Leu Pro Ala
    130                 135                 140

Ala Ala Pro Thr Pro Arg Ala Pro Gln Leu Val Ala Gln Leu Arg Gly
145                 150                 155                 160

Phe Leu Gly Asn Pro Ser Ala Ala Ala Leu Leu Arg Ser Arg Ala Trp
                165                 170                 175

Val Thr His Ala Pro Val Trp Asn Pro Arg Ser Leu Val Arg Pro Pro
            180                 185                 190

Val Asp Pro Ser Gly Asp Ile Ala Pro Thr His Ala Pro Arg Pro Pro
        195                 200                 205

Ala Gly Phe Pro Pro Asp Ala Gly Pro Ala Asp Ala Asp Pro Arg Ile
    210                 215                 220

Ser Phe Arg Glu Leu Ser Ala Ala His Leu Asn Asn Ala Ser Gly Thr
225                 230                 235                 240

Trp Leu Val Ala Ala Gly Leu Leu Arg Ala Pro Ser Ala Leu Val Tyr
                245                 250                 255

Arg Ser Pro Ser Ser Ala Thr Trp Pro Leu Ala Ile Trp Ala Thr Gly
            260                 265                 270

Glu Leu Ala Phe Gly Cys Asp Ala Ala Leu Val Arg Ala Arg Tyr Gly
        275                 280                 285

Leu Arg Phe Met Gly Leu Ser Leu Ser Met Arg Asp Ser Ala Pro Ala
    290                 295                 300

Glu Val Leu Val Val Pro Ala Ala Glu Thr Leu Ala Leu Ile Gly Pro
305                 310                 315                 320

Pro Ala Met Asn Glu Pro Leu Val Leu Pro Gly Pro Pro Pro Gly Lys
                325                 330                 335

Arg Tyr Arg Thr Phe Val Ile Gly Ser Val Val Asp Pro Arg Asn Val
            340                 345                 350

Ser Ala Ile Glu Ala Leu Arg Arg Ala Ala Arg Tyr Pro His Glu Asp
        355                 360                 365

Ala Gly His Glu His His Leu Ser Arg Ala Tyr Ala Glu Ile Phe Gly
    370                 375                 380

Glu Gly Pro Ser Val Glu Pro Gly Pro Arg Pro Pro Leu Phe Trp Arg
385                 390                 395                 400

Val Ser Ala Leu Leu Ala Thr Ser Gly Phe Ala Phe Thr Glu Thr Thr
                405                 410                 415

Arg Ala Arg Gly Met Leu Arg Leu Ser Asp Leu Val Asp Phe Leu Ala
            420                 425                 430

His Val Arg Val Ile Ala Asn Leu Ala Leu Arg Gly Ala Ala Gly Cys
        435                 440                 445

Ala Pro Gly Thr Pro Phe Ala Arg Ala Pro Leu Trp Ala Ala Pro Ala
    450                 455                 460

Arg Ala Glu Leu Glu Ser Arg Leu Gly Arg Leu Ala Ala Glu Ala Val
465                 470                 475                 480

Ala Arg Asp Gln Arg Leu Ser Ala Leu Ala Val Ala Tyr Gln Val Ala
                485                 490                 495

Phe Ala Leu Gly Asp Pro Ala Ile Ala Glu Ala Val Ala Pro Ser Ala
            500                 505                 510

Ala His Thr Leu Asp Thr Leu Tyr Ala Glu Phe Leu Arg Gly Arg Gly
        515                 520                 525

Leu Asp Ala Pro Ala Val Arg Arg Ala Leu Phe Tyr Ala Thr Ala Val
    530                 535                 540

Leu Arg Ala Pro Ala Glu Arg Gly Gly Ala Pro Ser Asp Ala Gln Val
545                 550                 555                 560

Thr Arg Gly Arg Arg Ser Leu Leu Leu Ala Ser Ala Met Cys Thr Ser
                565                 570                 575

Asp Val Ala Val Ala Thr His Thr Asp Leu Arg Asp Ala Leu Asp Arg
            580                 585                 590

Ser Asp His Arg Lys Thr Phe Phe Tyr Ala Pro Asp His Phe Ser Pro
        595                 600                 605

Cys Ala Ala Ser Leu Arg Phe Asp Leu Ala Glu Arg Ser Phe Val Met

610                 615                 620

Asp Thr Leu Ala His Thr Pro Arg Ser Asn Val Ser Val Glu Ala Met
625                 630                 635                 640

Ala Gln Lys Thr Gln Gly Val Ala Ser Ala Leu Thr Arg Trp Ala His
                645                 650                 655

Ala Asn Ala Leu Ile Arg Ala Phe Val Pro Glu Ala Ala Gln Thr Cys
            660                 665                 670

Ala Gly Pro Thr His Asn Ala Glu Pro Leu Val Val Leu Pro Val Thr
        675                 680                 685

Trp Asn Ala Ser Tyr Val Val Thr His Ala Pro Leu Pro Arg Gly Val
    690                 695                 700

Gly Tyr Arg Leu Ala Gly Val Asp Val Arg Arg Pro Leu Phe Leu Thr
705                 710                 715                 720

Tyr Leu Thr Glu Thr Cys Glu Gly Arg Thr Arg Glu Ile Glu Pro Lys
                725                 730                 735

Arg Leu Thr Arg Thr Glu Thr Arg Arg Asp Leu Gly Leu Val Gly Ala
            740                 745                 750

Val Phe Met Arg Tyr Thr Pro Ala Gly Glu Ile Met Ser Ala Leu Val
        755                 760                 765

Val Asp Ser Asp His Thr Gln Gln Gln Leu Ala Gly Gly Pro Leu Ala
    770                 775                 780

Gly Gly Val Asp Val Phe Val Ser Asp Val Pro Ser Thr Ala Leu Leu
785                 790                 795                 800

Leu Phe Pro Asn Gly Thr Val Ile His Leu Leu Ala Phe Asp Thr Leu
                805                 810                 815

Pro Leu Ala Thr Ile Thr Pro Gly Val Leu Ala Ala Ser Val Leu Gly
            820                 825                 830

Val Val Leu Ile Ala Ala Ala Ile Val Gly Leu Ala Arg Val Ala Trp
        835                 840                 845

Thr Cys Val Pro Ser Leu Trp Ser Arg Glu
    850                 855

<210>  6
<211>  224
<212>  PRT
<213>  Herpes B virus

<400>  6

Met Phe Leu Arg Ser Val Ser Ala Ala Pro Ser Val Val Ser Pro Ala
1               5                   10                  15

Ala Ser Pro Ser Pro Ser Pro Pro Val Glu Tyr Val Ile Arg Ser Val
            20                  25                  30

Ala Ala Arg Thr Val Gly Asp Ile Leu Lys Phe Ala Cys Leu Glu Leu
        35                  40                  45

Pro Ala Gly Gly Val Thr Trp Arg Tyr Glu Ala Pro Arg Ser Ile Asp
    50                  55                  60

Tyr Ala Arg Ile Asp Gly Ile Phe Leu Arg Tyr His Cys Pro Gly Leu
65                  70                  75                  80

Asp Thr Val Val Trp Asp Gly Lys Ala Gln Arg Ala Tyr Trp Val Asn
                85                  90                  95

Pro Phe Leu Phe Ala Ala Gly Phe Leu Glu Asp Leu Gly His Ala Leu
            100                 105                 110

Phe Pro Ala Asn Ala Leu Glu Thr Thr Thr Arg Phe Ala Leu Tyr Lys
        115                 120                 125

Glu Val Arg Leu Ala Leu Ala Ser Arg Ser Asp Ala Ala Ser Ser Thr
    130                 135                 140

Pro Val Pro Pro Gly Cys Val Asp Ala Glu Tyr Ser Arg Thr Arg Asp
145                 150                 155                 160

Cys Pro Asp Gly Arg Thr Pro Gly Ile Trp Asn Glu Pro Arg Ile Arg
                165                 170                 175

Arg Pro Phe Ser Ala Pro Asn Asp Glu Ala Ser Pro Gln Pro Gln Ser
            180                 185                 190

Leu Ala Pro Ala Pro Thr Pro Thr Pro Pro Gly Arg Thr His Glu Pro
        195                 200                 205

Ala Arg Lys Pro Arg Gly Asn Ala Thr Arg Thr Ala Arg Pro Arg Ala
    210                 215                 220

<210>  7
<211>  20
<212>  PRT
<213>  Herpes B virus

<400>  7

Cys Tyr Ser Arg Pro Leu Val Ser Phe Tyr Glu Glu Gly Gly Pro Leu
1               5                   10                  15

Val Glu Gly Gln
            20

<210>  8
<211>  42
<212>  PRT
<213>  Herpes B virus

<400>  8

Gly Ala Pro Arg Gly Ala Ala Ser Thr Pro Ala Gly Arg Pro Gly Ala
1               5                   10                  15

Ser Arg Pro Gly Gly Val Glu Arg Ala Asn Arg Thr Ala Ala Pro Ala
            20                  25                  30

Arg Gly Arg Gly Ser Ser Asn Gly Thr Gly
        35                  40

<210>  9
<211>  29
<212>  PRT
<213>  Herpes B virus

<400>  9

Gly Val Gly Val Tyr Val Arg Arg Ala Val Ala Arg Arg Arg Arg Val
1               5                   10                  15

Arg Thr Gly Arg Trp Ala Gly Glu Pro Ala Arg Arg Gly
            20                  25

<210>  10
<211>  36
<212>  PRT
<213>  Herpes B virus

<400>  10

Ala Val Ala Leu Ala Arg Val Pro Ala Gly Gly Gly Glu Tyr Val Pro
1               5                   10                  15

Val Glu Arg Ser Leu Thr Arg Val Asn Pro Gly Arg Phe Arg Gly Ala
            20                  25                  30

His Leu Ala Pro
        35

<210>  11
<211>  32
<212>  PRT
<213>  Herpes B virus

<400>  11

Leu Val Arg Arg Arg Ala Arg Ala Ala Gly Lys His Val Arg Leu Pro
1               5                   10                  15

Glu Leu Leu Asp Glu Gly Pro Gly Pro Ala Arg Arg Gly Ala Pro Tyr
            20                  25                  30

<210>  12
<211>  24
<212>  PRT
<213>  Herpes B virus

<400>  12

Ala Arg Asp Gln Arg Leu Ser Ala Leu Ala Val Ala Tyr Gln Val Ala
1               5                   10                  15

Phe Ala Leu Gly Asp Pro Ala Ile
            20